# EUROPEAN PATENT SPECIFICATION

(11) **EP 3 952 932 B1**
(45) Date of publication and mention of the grant of the patent: **20.12.2023**
(21) Application number: 20714006.2
(22) Date of filing: 12.03.2020
(51) Int. Cl.: A61L 17/10, A61L 27/18, A61L 27/54, A61L 31/06, A61L 31/16, B33Y 10/00, B33Y 80/00, D01F 1/00, C01B 32/198, B29C 64/106, B33Y 70/00, D01D 5/06, D01F 1/10, D01F 6/62

(54) **COMPOSITIONS AND METHODS FOR 3D PRINTED FIBROUS SCAFFOLDS WITH ANTIMICROBIAL PROPERTIES INCORPORATING GRAPHENE OXIDE AND POLY(E-CAPROLACTONE)**
ZUSAMMENSETZUNGEN UND VERFAHREN FÜR 3D-GEDRUCKTE FASERGERÜSTE MIT ANTIMIKROBIELLEN EIGENSCHAFTEN, DIE GRAPHENOXID UND POLY(E-CAPROLACTON) ENTHALTEN
COMPOSITIONS ET PROCÉDÉS POUR ÉCHAFAUDAGES FIBREUX IMPRIMÉS EN 3D DOTÉS DE PROPRIÉTÉS ANTIMICROBIENNES INCORPORANT DE L'OXYDE DE GRAPHÈNE ET DE LA POLY(E-CAPROLACTONE)

(30) Priority: 12.03.2019 PT 2019115361
(43) Date of publication of application: 16.02.2022
(73) Proprietor: Instituto de Engenharia Biomédica (INEB), 4200-135 Porto (PT)
(72) Inventor: FERREIRA MELO, Sofia, 4200-135 Porto (PT); DE CASTRO GONÇALVES ALMADA LOBO, Inês, 4200-135 Porto (PT); CARVALHEIRA NEVES, Sara, 4200-135 Porto (PT)
(74) Representative: Ferreira Pinto, Francisca
(86) International application number: PCT/PT2020/050010
(87) International publication number: WO 2020/185106

(56) References cited:
- CN-A- 108 714 234
- PUPPI DARIO ET AL: "Levofloxacin-loaded star poly([epsilon]-caprolactone) scaffolds by additive manufacturing", JOURNAL OF MATERIALS SCIENCE: MATERIALS IN MEDICINE, SPRINGER NEW YORK LLC, UNITED STATES, vol. 27, no. 3, 12 January 2016 (2016-01-12), pages 1-11, XP035915849, ISSN: 0957-4530, DOI: 10.1007/S10856-015-5658-1 [retrieved on 2016-01-12]
- DUYGU EGE ET AL: "Graphene Oxide/Polymer-Based Biomaterials", ADVANCED ENGINEERING MATERIALS., vol. review GO19, no. 12, 14 September 2017 (2017-09-14), page 1700627, XP055701279, DE ISSN: 1438-1656, DOI: 10.1002/adem.201700627

## Description

### Technical field of the invention

The present invention refers to 3D methods to produce a Poly(e-caprolactone) - PLC - and Graphene Oxide (GO) scaffold with bactericidal properties against bacteria that cause infections in patients implanted with medical devices. As such, the present invention relates to the technical field of polymers, particularly to PLC/GO composites, to medical biotechnology, and specifically to medical devices with concurrent bactericidal properties.

### State of the art

One of the main challenges associated to implantable devices is to find efficient strategies to face bacterial adhesion and consequent infection [1]. Device colonization by bacteria can lead to its malfunction, as it may result in biofilm formation at the implantation site [2]. This represents a serious health problem, worsened by growing antibiotic resistance, and causing the loss of the implanted device or even sepsis [3]. This has been subject of extensive research, but the desirable successful prevention or effective solution are yet to be achieved. Common organisms associated to polymeric meshes infection are *Staphylococcus* spp. [4,5], with *Staphylococcus epidermidis* on the leading positions [6], but also Gram-negative *Enterobacteriacea,* such as the rod-shaped *Escherichia coli. These are both* biofilm-forming bacteria which produce extracellular polysaccharides when proliferating on a surface [7], enhancing their survival efficiency. *S. epidermidis* has a naturally high resistance to antimicrobials, which generates great concerns. *E. coli* also resists to antibiotics such as penicillin, since the outer membrane surrounding the cell wall provides an additional barrier. Bacterial adhesion to device's surface is not a one-time phenomenon, but rather an evolving process. Initially, there is a rapid attachment to the surface, mediated either by nonspecific factors (such as surface tension, hydrophobicity, and electrostatic forces) or by specific adhesins [8], followed by an accumulation phase, during which bacteria adhere to each other and form the biofilm [9]. Alternatives to antibiotics are being thoroughly explored, and carbon-derived materials are receiving growing attention [10]. Since graphene's (G) first isolation in 2004 [11], its derivatives have been developed and investigated, commonly conjugated with polymers to produce composites or used to modify their surface [12-14]. In 2010, the antibacterial properties of graphene-based materials (GBMs) were explored for the first time [15], leading to a growing number of reports that describe some GBMs as antimicrobial nanomaterials [16-21]. The interaction between GBMs and biological systems has also been studied [22-25], giving some insights regarding their effect on different types of organisms. Nevertheless, these interactions need to be explored in more detail regarding GBMs immobilized in polymeric matrices, since their characteristics may vary. Antimicrobial properties, for instance, are known to be different when comparing GBMs in suspension with GBMs immobilized on a surface [21,26-28]. Moreover, direct physical contact of bacteria with GBMs at a surface (either with sharp edges or basal planes) is a requirement for GBMs-containing biomaterials to have an antibacterial action, with no effect being observed when no direct contact is established [28,29]. Smaller and more oxidized forms of GBMs have been associated with higher biocompatibility towards mammalian cells [30]. Furthermore, stronger bactericidal properties have been described in oxidized forms of graphite (GO) and graphene nanoplatelets (GNPs) [18,21,31]. It is described that the orientation and exposure of GO sheets on the fibers' surface are important parameters, being essential factors for antibacterial properties [28]. To create 3D-structured scaffolds in tissue engineering, combination of additive manufacturing (AM) with wet-spinning has been described [32-34]. Poly(e-caprolactone) (PCL) is currently among the most popular synthetic polymers used [35]. PCL fibers assembled into random or organized 3D structures have been broadly studied in the scope of tissue engineering approaches [36-39]. PCL is highly appealing due to its physical-chemical and mechanical characteristics [40,41], and non-toxic degradation products. It received Food and Drug Administration (FDA) approval and European Conformity (CE) marking for a number of drug delivery and medical device applications [40]. Besides, this polymer presents additional advantages, namely availability, relatively low cost, suitability for modification [42], and a relatively long biodegradation time, which makes it widely used also in long-term implants [43] . Improvements in PCL performance have been attempted through its modification, namely by adding new components like GO [39]. Although few studies are available [44], GO incorporation in PCL matrices has led to improvements in terms of hydrophilicity, mechanical and thermal properties, and biocompatibility [45-47].

However, the ability of these methods to produce PCL/GO matrices with antibacterial properties cannot be extrapolated and the methods chosen for fiber-based composite antimicrobial scaffolds production must be such that to enhance GO exposure.

This is because GO antibacterial effects have been associated with either induced oxidative stress or bacteria physical disruption, thus requiring GO exposure in the fiber surface, to contact with bacteria.

On one hand, fibers need to be wide enough to incorporate GO sheets presenting diameters ranging from 2 to 10 µm. This excludes nanometric fibers production techniques, such as electrospinning [46,48-50].

On the other hand, GO exposure in composites produced by melt-dependent techniques is known to be difficult to obtain [28], since GO sheets are usually covered or encapsulated by the polymer in the obtained fibers [51].

Thus, the methods known in the art for incorporation of GO in PCL matrices do not solve the problem of controlling the orientation and exposure of GO sheets on the fibers surface so as to achieve antibacterial properties Further methods for preparing PCL fibers are described in the following. PUPPI DARIO ET AL: "Levofloxacin-loaded starpoly(epsilon-caprolactone) scaffolds by additive manufacturing" SCIENCE:MATERIALS IN MEDICINE, vol. 27, no.3, 12 January 2016 (2016-01-12), pages 1 - 11, XP03:S10856-0155658-1 discloses a fibrous scaffold comprising the antibiotic levofloxacin, acetone, PCL and EtOH. These fibers are prepared by a process comprising a) dissolving PCL in acetone to obtain a homogeneous solution (20% w/v); b) adding the antibiotic (LF/PCL weight ratio of 0.013 or 0.065:1) to said solution; c) loading the mixture in a syringe; d) extruding the PCL/LF mixture directly into a bath of ethanol; e) drying the scaffolds in a desiccator. It

DATABASE WPI week 201880 Thomson Scientific, London GB, AN2018-86243K & CN108714234A October 2018 (2018-10-30)discloses an antibacterial fibrous membrane comprising GO, ethanol, PCL and CH2Cl2. The fibers are prepared by a process comprising: a) preparing a homogeneous solution of GO and EtOH; b) preparing a solution of PCL in CH2Cl2; c) mixing said solutions together (electrospinning solution A) ; d) preparing a sodium alginate and PVA solution in water (electrospinning solution B); e) placing solutions A and B in an electrospinning machine and coaxial electrospinning said polymeric solutions to obtain a fiber with an inner core of alginate/PVL and an outer layer of PCL/GO; f) extruding said fibers into a coagulation bath comprising CaCl2 and ethanol, washing with ethanol and drying.

These facts are disclosed in order to illustrate the technical problem addressed by the present disclosure.

### Brief description of the invention

Disclosed are the parameters of a composition of Poly(e-caprolactone) - PLC - and Graphene Oxide (GO) for use in killing bacteria that cause infections in patients implanted with medical devices, for example Staphylococcus epidermidis and *Escherichia coli.* This composition can be employed in a method for 3D printing of PLC/GO fibers and fibrous scaffolds by additive manufacturing and wet spinning. In the above-mentioned compositions and method the PLC/GO concentrations and the fiber diameter are such that GO sheets are incorporated but at the same time exposed at the polymer surface, coffering bactericidal properties to the material, while keeping biocompatibility.

Thus, also disclosed is a composition of Poly (e-caprolactone) and Graphene Oxide for use in killing bacteria that cause infections in patients implanted with medical devices comprising: a solvent consisting of Tetrahydrofuran (THF); Graphene Oxide (GO) at final concentration between 5% and 7.5% (w/w), most preferably 5% (w/w); Poly(e-caprolactone) -PCL- at final concentration between 7.5% and 15% (w/v), most preferably 7.5% (w/v);A coagulation non-solvent consisting of ethanol.

It is further disclosed that the said bacteria that causes infections in patients implanted with medical devices comprises for example *Staphylococcus epidermidis* and *Escherichia coli.*

It is further disclosed that the said Poly(e-caprolactone) and Graphene Oxide polymer is constructed into a scaffold. It is further disclosed that the said Poly(e-caprolactone) and Graphene Oxide polymer is constructed into a fibrous scaffold.

It is further disclosed that a fibrous scaffold of Poly(e-caprolactone) and Graphene Oxide for killing bacteria that cause infections in patients implanted with medical devices comprising: a solvent consisting of Tetrahydrofuran (THF); Graphene Oxide (GO) at final concentration between 5% and 7.5% (w/w), most preferably 5% (w/w); Poly (e-caprolactone) -PCL- at final concentration between 7.5% and 15% (w/v), most preferably 7.5%; a coagulation non-solvent consisting of ethanol.

It is further disclosed that the fibers have a diameter from 50 to 100 µm, most preferably of 100 µm.

It is further disclosed that the said bacteria that causes infections in patients implanted with medical devices comprises for example *Staphylococcus epidermidis* and *Escherichia coli.*

It is further disclosed that the said composition or scaffold is comprised in a medical device, implanted in an animal or the human body.

It is further disclosed that the said medical device is selected from the list consisting of surgical sutures, 3D scaffolds for tissue engineered implants as well as other non-limiting examples of implantable medical devices such as a stent, a vascular implant, a dental implant, and a bone implant.

The present invention refers to a method for constructing a fibrous scaffold of Poly(e-caprolactone) and Graphene Oxide by additive manufacturing and wet-spinning of comprising the steps of:
a) Dispersing graphene oxide (GO) in Tetrahydrofuran (THF), solvent at a final concentration of 5% w/w to 7.5% w/w, most preferably 5% w/w;
b) Dissolving Poly(e-caprolactone) - PLC- in the previous mixture at a concentration of 7.5% to 15%, most preferably 7.5%
c) Loading the previous mixture into a glass syringe or a 3D printer head
d) Extruding the THF/PCL/GO mixture into a coagulation bath consisting of ethanol
e) Rinsing PCL/GO scaffolds with ethanol and drying the scaffolds
, as described in claims 1 and 2.

In another embodiment of the present invention's method, the said extrusion of THF/PCL/GO mixture is performed layer-by-layer in a 3D plotting machine or a 3D printer, as described in claim 3.

### Detailed description of the Invention

Disclosed are the parameters of a composition of Poly(e-caprolactone) - PLC - and Graphene Oxide (GO) for use in killing bacteria that cause infections in patients implanted with medical devices, for example Staphylococcus epidermidis and *Escherichia coli.* This composition can be employed in a method for 3D printing of PLC/GO fibers and fibrous scaffolds by additive manufacturing and wet spinning. In the above-mentioned compositions and method the PLC/GO concentrations and the fiber diameter are such that GO sheets are incorporated but at the same time exposed at the polymer surface, coffering bactericidal properties to the material, while keeping biocompatibility.

The GO is prepared according to the Modified Hummer's Method (MHM) [52] adjusting reagents volume/mass for larger scale production (2000 mL flasks). Briefly, 320 mL of H2SO4 (VWR, Germany) are mixed with 80 mL of H3PO4 (Chem-Lab, Belgium) in a 4:1 ratio, and stirred at room temperature (RT) for an improved oxidation. 8 g of graphite (carbon graphite micropowder, American Elements, purity above 99%, diameter between 7 and 11 µm) are added to this solution and then cooled down to 0 °C using an ice bath before gradual addition of 48 g of KMnO4 (JMGS, Portugal). The solution is heated up to 35 °C and stirred for 2 hours. After lowering the temperature to 0 °C, 1200 mL of distilled water are slowly added. This is followed by careful addition of H₂O₂ 35% (WWR, Germany) until oxygen release stops. After overnight resting, the solution is decanted to separate the solid deposit from the acidic supernatant. The remaining product is washed with dH₂O and this aqueous solution is centrifuged at 4000 rpm for 20 minutes at RT. This step is repeated until the washing water pH is equal to dH₂O pH. By the end of this process, sonication is performed for 6h to exfoliate the oxidized material into graphene oxide.

To prepare PCL (obtained from Sigma-Aldrich, average Mn 80000 g mol) incorporating GO, an appropriate solvent needs to be used. Thus, the first parameter for the present disclosure's composition was the selection of an effective PCL solvent that efficiently disperses GO. The dispersion homogeneity and stability of GO was evaluated in different organic solvents, commonly considered potential solvents for PCL, namely: chloroform (VWR, Germany), acetone (JMGS, Portugal) and tetrahydrofuran (THF, VWR, Germany). GO aqueous dispersion is centrifuged for 1h at RT and 15000 rpm and the supernatant water is decanted. GO was resuspended in each of the solvents and PCL dissolution tests were carried out by using a polymer concentration of 15% w/v with permanent magnetic stirring (300 rpm) at RT. GO dispersion in chloroform was not achieved, revealing large GO aggregates and an absence of GO affinity to this solvent. On the contrary, acetone and THF exhibited a similar behavior, producing dispersions with perfectly dispersed GO, stable even after 10 days. All solvents tested were able to dissolve PCL, although this dissolution was only partial when acetone was used.

THF was therefore demonstrated to be able to perfectly disperse GO while efficiently dissolving PCL, and thus THF is the optimal solvent (Table 1).

**Table 1. Solvents tested to disperse GO and to dissolve PCL.**

| Solvent | GO Dispersion | PCL dissolution |
|---|---|---|
| Chloroform | GO aggregates formation; poor GO dispersion after ultrasonication | Complete dissolution after 3 hours; clear appearance |
| Acetone | Homogeneous GO dispersion after ultrasonication | Partial dissolution overnight; clear appearance when heated |
| Tetrahydrofuran | Homogeneous GO dispersion after ultrasonication | Complete dissolution overnight; clear appearance |

A THF/polymer mixture was then used to evaluate the optimal solvent/non-solvent combination. PCL non-solvents, namely isopropanol (VWR, Germany) and ethanol (VWR, Germany), were tested, to assess the best coagulation bath to obtain the polymer filaments.

Ethanol showed compatibility with good design definition and relatively fast polymer fiber precipitation during solvent/nonsolvent exchange. Moreover, ethanol excess is easy to eliminate from the scaffolds through evaporation. Isopropanol was also tested as a coagulation bath and although a similar performance was observed when compared to ethanol, a slightly worse fiber definition was achieved. As such, ethanol was selected as the optimal composition component for the coagulation bath (Table 2).

**Table 2. Solvent/non-solvent combinations tested for the polymer fiber coagulation.**

| Solvent | Non-solvent | Observation |
|---|---|---|
| Tetrahydrofuran | Ethanol | Several layers could be printed; fiber diameter around 100pm; good fiber definition |
| Tetrahydrofuran | Isopropanol | Several layers could be printed; fiber diameter around 100pm; fair fiber definition |

Different PCL and GO concentrations were tested, ranging from 7.5% to 15% w/v (weight of PCL per volume of solvent) and from 0% to 10% w/w (weight of GO per weight of PCL), respectively. Results of the assessment of the optimal PLC/GO combination is shown in Table 3.

**Table 3. Assessment of the optimal PCL and GO concentrations.**

| PCL concentration (w/v) | GO concentration (w/w) | Observations |
|---|---|---|
| 7,5% | 0% | Suitable viscosity; no clogging |
| 7,5% | 5% | Suitable viscosity; occasional clogging; fibers showed wider combs (cross section) in the presence of GO |
| 7.5% | 7,5% | High viscosity; occasional clogging; fibers showed wider combs in the presence of GO |
| 7,5% | 100 | Very high viscosity; frequent clogging; GO aggregates observed |
| 150 | 0% | High viscosity; no clogging |
| 150 | 5% | High viscosity; occasional clogging; fibers showed wider combs in the presence of GO |
| 15% | 7,5% | Very high viscosity; constant clogging; GO aggregates observed; not printable. |
| 150 | 10% | Very high viscosity; constant clogging; GO aggregates observed; not printable. |

For the 7.5% (w/v) PCL concentration, GO concentrations between 0% and 10% (w/w) were tested, although the highest GO concentration hindered the solution extrusion process, causing frequent needle clogging.

Regarding the 15% (w/v) PCL solution, GO concentration could not be increased above 5% (w/w), since higher concentrations affected the printing process due to constant clogging observed. Moreover, major effects in fiber morphology were observed.

A PCL concentration of 7.5% (w/v) and a range of GO concentrations from 5% to 7.5% (w/w) provided therefore the optimal conditions, allowing the production of porous fibers with different amounts of GO loading.

Thus, in the present invention GO is dispersed in a solvent consisting of THF at final concentrations between 5% (w/w) and 7.5% (w/w). PCL is then dissolved overnight at RT in these dispersions to a final polymer concentration of 7.5% (w/v). Polymer filaments are obtained in a non-solvent coagulation bath consisting of ethanol.

Given their suitable viscosity, the above-mentioned compositions allow the development of a method for fiber-based production of composite scaffolds employing a combination of additive manufacturing and wet-spinning in which a dispensing tip is fed with a polymeric composition solution and submerged in a non-solvent of the polymer that causes its precipitation and filament formation.

In one embodiment, compositions are loaded into a glass syringe with a needle with internal diameter of 184 µm (28G) that is used for scaffold plotting and the THF/GO/PLC solution is extruded into an ethanol coagulation bath using a syringe pump.

When printed, PCL and PCL/GO scaffolds are rinsed 3 - 5 times with ethanol, dried in a fume hood and cut using a 4 mm diameter stainless steel biopsy puncher (Integra). Scaffolds are sterilized with ethylene oxide following the established protocol for medical material sterilization (Millex).

Thus, the present invention refers to a method for constructing PLC/GO fibers by additive manufacturing and wet spinning of comprising the steps of:
a) Dispersing graphene oxide (GO) in Tetrahydrofuran (THF), solvent at a final concentration of 5% to 7.5% (w/w), most preferably 5% (w/w);
b) Dissolving Poly(e-caprolactone) - PLC- in the previous mixture at a concentration of 7.5% to 15% (w/v), most preferably 7,5% (w/v);
c) Loading the previous mixture into a glass syringe or a 3D printer head;
d) Extruding the THF/PCL/GO mixture into a coagulation bath consisting of ethanol;
e) Rinsing PCL/GO scaffolds with ethanol and drying the scaffolds.

In another embodiment of the present invention's method, the layer-by-layer fabrication of the scaffolds can also be performed using a 3D plotting machine (such as a xyz plotter), adapting the setup previously described by Neves et al. [53]. Printing parameters such as flow rate (F) of the syringe pump and deposition speed (Vdep) of the xyz plotter (defined as relative percentage to the plotter firmware parameters), can be adjusted, ranging from 0.5 mL/h to 1.0 mL/h and from 50% to 120%, respectively

In one embodiment the printing parameters are defined as F - 0.5 mL/h and Vdep - 80%, which showed to be the optimal option, given the ideal compromise between good fiber definition and time consumption.

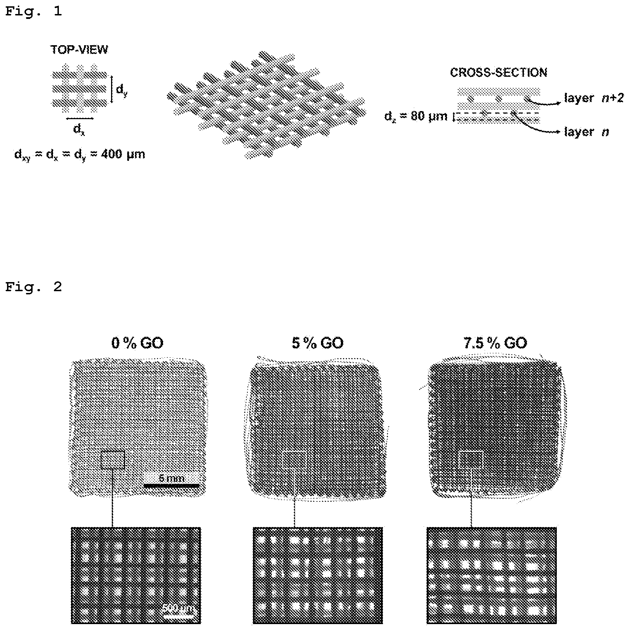

The 3D design can be varied and optimized in terms of xyz inter-fiber distances and staggering. The best fit for well-defined, precisely spaced structures is achieved by xy distances ranging from 200 µm to 400 µm, z-steps from 20 µm to 80 µm, and staggering between layers from 50 µm to 200 µm. A final 3D design of the scaffolds is displayed in Figure 1 (virtual scheme), and Figure 2 (Stereomicroscope images) including top-view and cross-section schemes.

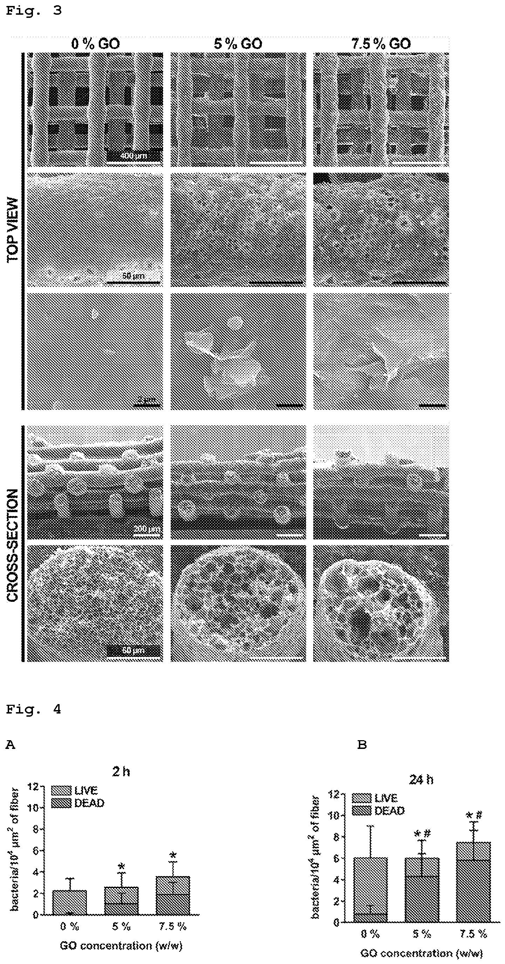

Scanning electron microscopy (SEM) observation of the scaffolds corroborates the precise plotting of filaments, showing the staggering between layers, visible in both top view and cross-section images (Figure 3).

Fibers' average diameter can be measured along different fibers, 0% GO scaffolds presenting average diameters of 107 ± 11 µm, 5% GO scaffolds of 102 ± 9 µm and 7.5% GO scaffolds of 103 ± 13 µm. From the SEM analysis, it is also possible to confirm GO incorporation in the fibers and its exposure. Top views of the fibers surface show GO exposure for both 5% and 7.5% GO concentrations (Figure 3). GO sheets protrude from the surface, creating a wrinkled topography, with surface roughness and irregularity increasing when GO is incorporated (Figure 3).

Focusing on the cross-sections, it is possible to observe that the inner porosity of the fibers changes with the presence of GO within the PCL matrix. The size and the irregularity of the combs increase in a direct proportion to the amount of incorporated GO. SEM analysis allows to demonstrate that GO/PCL scaffolds with a 3D network of macropores is successfully fabricated and GO exposure at the fibers surface is achieved in 5% and 7.5% GO scaffolds.

Thus, employing the above-mentioned compositions and method of additive manufacturing with wet-spinning it is possible to obtain PLC/GO fiber production with a 50 - 150 µm diameter range (Figure 3). This is sufficiently wide to incorporate GO sheets, while narrow enough so that GO sheets are exposed in order to confer GO-mediated antibacterial activity to the scafold's fibers (Figure 3).

Regarding the antimicrobial potential of the developed structures, namely antibacterial activity towards Staphylococcus epidermidis, the effect of GO presence is noticed after a relatively short period (2h), apparently being bactericidal (Figure 4).

PCL/GO scaffolds antibacterial effect assessed after 2h and 24h incubation with S. epidermidis shows that in the 2h incubation assay, higher amounts of dead bacteria are found in 5% GO (1.10 ± 0.93 bacteria/10⁴µm²) and 7.5% GO (1.94 ± 1.10 bacteria/10⁴µm²) comparing to 0% GO scaffolds (0.07 ± 0.10 bacteria/10⁴µm²) (p = 0.0014 and p < 0.0001, respectively) (Figure 4).

The incorporation of GO promotes bacterial death, with the percentages of dead bacteria increasing from only 2.9% in 0% GO to 41.8% in 5% GO and 53.8% in 7.5% GO (Figure 4A).

In a 24h incubation assay presented in the bactericidal effect of GO-containing scaffolds is confirmed and the death rates are higher comparing to the ones obtained after 2h incubation, being 13.7% in 0% GO, and reaching 71.9% in 5% GO and 77.8% in 7.5% GO scaffolds (Figure 4B). The number of dead bacteria was again higher in 5% and 7.5%, when compared to 0% GO scaffolds (p < 0.0001 in both concentrations); (Figure 4B).

Moreover, the number of live bacteria that adhered to the scaffolds was significantly lower in GO-containing scaffolds (p = 0.0001), with only around 1.7 bacteria/10⁴µm2), comparing to around 5.3 bacteria/10⁴µm² in PCL with 0% GO scaffolds. These interesting results also demonstrate that while the number of live bacteria increases in PCL with 0% GO scaffolds from 2h to 24h, this does not occur in GO-containing scaffolds. In 5% and 7.5% GO, the number of live bacteria is maintained over the 24h, revealing the bactericidal effect of these scaffolds over time.

GO appeared to act as a killing agent, rather than contributing for the formation of an antifouling surface. Data revealed that a GO concentration of 5% seems to be enough to produce the desired bactericidal effects in all the performed assays, since no statistically significant differences were found between this concentration and the highest one (7.5% GO).

Thus, an antibacterial effect is unravelled, possibly towards different bacteria, including but not limited to *S. epidermidis,* one of the most commonly found microorganism in implant-associated infections.

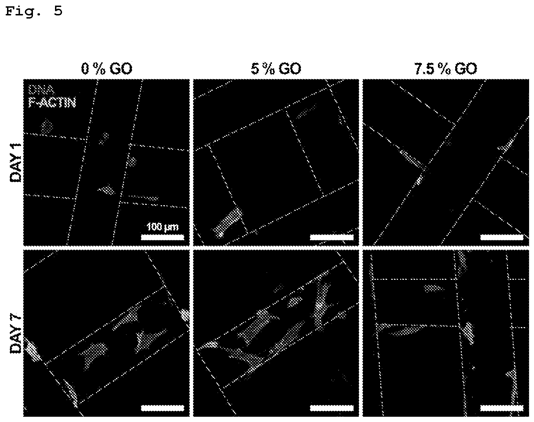

Regarding the in vitro biocompatibility, HFF-1 human fibroblast cell line spread morphology after 7 days of culture in all conditions indicates that scaffolds are noncytotoxic (Figure 5).

Since bacterial infection represents a constant threat when implantable medical devices are used, our data suggests promising performance of PCL/GO scaffolds in several biomedical applications, namely as surgical sutures or 3D scaffolds for tissue engineering, in which PCL alone is currently used.

Thus, also disclosed are medical devices comprising the above-mentioned compositions, fibers and fibrous scaffolds that are implanted in an animal or the human body such as surgical sutures, 3D scaffolds for tissue engineered implants as well as other non-limiting examples of implantable medical devices such as a stent, a vascular implant, a dental implant, and a bone implant.

In conclusion, wet spinning combined with additive manufacturing allowed the production of well-defined PCL/GO fibrous scaffolds with average fiber diameters of 100 µm. A concentration of 5% GO was enough to expose GO sheets at the surface of the composite fibers. Antimicrobial properties of composite PCL/GO 3D-organized fibrous scaffolds were assessed for the first time, revealing GO time-dependent bactericidal effect and an increase in death rate from about 14% in neat PCL scaffolds to nearly 80% in composite scaffolds with 7.5% GO, after 24h of contact. In vitro biocompatibility evaluation showed that PCL and composite PCL/GO scaffolds allowed human fibroblasts adhesion and spreading along the fibers during 7 days of culture. As such, GO-containing fibrous scaffolds developed herein promoted bacterial death, while allowing human cells adhesion. These features demonstrate the parameters of GO incorporation in polymer fibrous scaffolds for antimicrobial properties on implanted device-associated infections.

The above described embodiments are combinable.

### Brief Description of the Figures

**Figure 1**: 3D model of the produced scaffolds.
   Top view (where dx and dy are displayed) and cross-section view (where dz is shown and the staggering between layers is visible)
**Figure 2****: Stereomicroscope images of obtained PCL scaffolds with 0%, 5% and 7.5% GO.**
   The lower lane represents zoomed regions of the upper lane images. Scale bar: top - 5 mm; bottom (zoom) - 500 µm. PCL and composite PCL/GO fibrous scaffolds with approximately 1.2 cm x 1.2 cm were printed, according to the 3D model previously disclosed in Fig. 1. The obtained 0% GO scaffolds (pristine PCL) are white, and the incorporation of GO within the polymeric matrix can be verified by the change in color as GO-containing scaffolds present a light brown (for 5% GO) and dark brown (for7.5% GO) color. As can be observed, fibers are well defined and precisely plotted following the virtual design
**Figure 3****: SEM analysis of the top views and cross-sections of PCL scaffolds with 0%, 5% and 7.5% GO.**
   Scale bar (from the top to the bottom row): top view - 400 µm, 50 µm, 2 µm; bottom - 200 µm, 50 µm.
**Figure 4****: Antibacterial properties of PCL/GO fibrous scaffolds**
   *S. epidermidis* adhesion to PCL scaffolds with 0%, 5% and 7.5% GO, after 2h and 24h incubation in 10% v/v plasma supplemented TSB. Bacteria were stained with the LIVE/DEAD Backlight kit (ThermoFisher) and counted by confocal microscopy. Stacked bars graph with live and dead bacteria counting *per* 10⁴µm² of fiber is displayed. Statistically significant differences on the number of live and dead (*) bacteria compared to 0% GO scaffolds are indicated on top of the stacked bars (p = 0.05; non-parametric Kruskal-Wallis test) .
**Figure 5****: In *vitro* biocompatibility assessment**
   Representative confocal microscopy images of HFF-1 human fibroblasts interaction with PCL scaffolds with 0%, 5% and 7.5% GO, after 1 day and 7 days of culture. Cells are identified by staining the nuclei with DAPI (DNA) and cytoskeleton (F-actin staining). The scaffold fibres can be identified by the faint-clear dashed lines. Images represent single plane projections of a 150 µm height z-stack. Scale bar: 100 µm.

### References

[1] VanEpps JS, Younger JG. Implantable Device-Related Infection SHOCK 2016;46:597-608. doi:10.1097/SHK.0000000000000692.
[2] Darouiche RO. Device-Associated Infections: A Macroproblem that Starts with Microadherence. Clin Infect Dis 2001;33:1567-72. doi:10.1086/323130.
[3] Rimondini L, Fini M, Giardino R. The microbial infection of biomaterials: A challenge for clinicians and researchers. A short review. J Appl Biomater Biomech 2005;3:1-10.
[4] Falagas ME, Kasiakou SK. Mesh-related infections after hernia repair surgery. Clin Microbiol Infect 2005;11:3-8. doi:10.1111/j.1469-0691.2004.01014.x.
[5] Akyol C, Kocaay F, Orozakunov E, Gene V, Kepenekci Bayram I, Cakmak A, et al.Outcome of the patients with chronic mesh infection following open inguinal herniarepair. J Korean Surg Soc 2013;84:287-91. doi:10.4174/jkss.2013.84.5.287.
[6] Guillaume O, Pérez-Tanoira R, Fortelny R, Redl H, Moriarty TF, Richards RG, et al.Infections associated with mesh repairs of abdominal wall hernias: Are antimicrobialbiomaterials the longed-for solution? Biomaterials 2018;167:15-31. doi:10.1016/j.biomaterials.2018.03.017.
[7] O'Toole G, Kaplan HB, Kolter R. Biofilm Formation as Microbial Development. Annu RevMicrobiol 2000;54:49-79. doi:10.1146/annurev.micro.54.1.49.
[8] Rupp ME, Ulphani JS, Fey PD, Bartscht K, Mack D. Characterization of the importance of polysaccharide intercellular adhesin/hemagglutinin of Staphylococcus epidermidis in the pathogenesis of biomaterial-based infection in a mouse foreign body infection model. Infect Immun 1999;67:2627-32.
[9] Galdbart J, Allignet J, Tung H, Rydèn C, El Solh N. Screening for Staphylococcus epidermidis Markers Discriminating between Skin-Flora Strains and Those Responsible for Infections of Joint Prostheses. J Infect Dis 2000;182:351-5. doi:10.1086/315660.
[10] Al-Jumaili A, Alancherry S, Bazaka K, Jacob M V. Review on the antimicrobial properties of Carbon nanostructures. Materials (Basel) 2017;10:1-26. doi:10.3390/ma10091066.
[11] Novoselov KS, Geim AK, Morozov S V, Jiang D, Zhang Y, Dubonos S V, et al. Electric Field Effect in Atomically Thin Carbon Films. Science 2004;306:666-9. doi:10.1126/science.1102896.
[12] Feng R, Guan G, Zhou W, Li C, Zhang D, Xiao Y. In situ synthesis of poly(ethylene terephthalate)/graphene composites using a catalyst supported on graphite oxide. J Mater Chem 2011;21:3931. doi:10.1039/c0jm03600e.
[13] Jakus AE, Secor EB, Rutz AL, Jordan SW, Hersam MC, Shah RN. Three-dimensional printing of high-content graphene scaffolds for electronic and biomedical applications. ACS Nano 2015;9:4636-48. doi:10.1021/acsnano.5b01179.
[14] Das TK, Prusty S. Graphene-Based Polymer Composites and Their Applications. Polym Plast Technol Eng 2013;52:319-31. doi:10.1080/03602559.2012.751410.
[15] Hu W, Peng C, Luo W, Lv M, Li X, Li D, et al. Graphene-Based Antibacterial Paper. ACS Nano 2010;4:4317-23. doi:10.1021/nn101097v.
[16] Tashan H, Darani KK, Yazdian F, Omidi M, Sheikhpour M, Sadeghizadeh M, et al. Antibacterial Properties of Graphene Based Materials: Emphasis on Molecular Mechanisms, Surface Engineering and Size of Sheets. Mini Rev Org Chem 2018;15. doi:10.2174/1570193X15666180712120309.
[17] Pham VTH, Truong VK, Quinn MDJ, Notley SM, Guo Y, Baulin VA, et al. Graphene Induces Formation of Pores That Kill Spherical and Rod-Shaped Bacteria. ACS Nano 2015;9:8458-67. doi:10.1021/acsnano.5b03368.
[18] Liu S, Zeng TH, Hofmann M, Burcombe E, Wei J, Jiang R, et al. Antibacterial Activity of Graphite, Graphite Oxide, Graphene Oxide, and Reduced Graphene Oxide: Membrane and Oxidative Stress. ACS Nano 2011;5:6971-80. doi:10.1021/nn202451x.
[19] Kim TI, Kwon B, Yoon J, Park I-J, Bang GS, Park Y, et al. Antibacterial Activities of Graphene Oxide-Molybdenum Disulfide Nanocomposite Films. ACS Appl Mate Interfaces 2017;9:7908-17. doi:10.1021/acsami.6b12464.
[20] Gurunathan S, Woong Han J, Abdal Daye A, Eppakayala V, Kim J. Oxidative stressmediated antibacterial activity of graphene oxide and reduced graphene oxide in Pseudomonas aeruginosa. Int J Nanomedicine 2012;7:5901. doi:10.2147/IJN.S37397.
[21] Perreault F, De Faria AF, Nejati S, Elimelech M. Antimicrobial Properties of Graphene Oxide Nanosheets: Why Size Matters. ACS Nano 2015;9:7226-36. doi:10.1021/acsnano.5b02067.
[22] Yang Y, Asiri AM, Tang Z, Du D, Lin Y. Graphene based materials for biomedical applications. Mater Today 2013;16:365-73. doi:10.1016/J.MATTOD.2013.09.004.
[23] Pinto AM, Gonçalves IC, Magalhães FD. Graphene-based materials biocompatibility: A review. Colloids Surfaces B Biointerfaces 2013;111:188-2.doi:10.1016/j.colsurfb.2013.05.022.
[24] Pang L, Dai C, Bi L, Guo Z, Fan J. Biosafety and Antibacterial Ability of Graphene and Graphene Oxide In Vitro and In Vivo. Nanoscale Res Lett 2017;12:564. doi:10.1186/s11671-017-2317-0.
[25] Chen Q, Mangadlao JD, Wallat J, De Leon A, Pokorski JK, Advincula RC. 3D printing biocompatible polyurethane/poly(lactic acid)/graphene oxide nanocomposites:Anisotropic properties. ACS Appl Mater Interfaces 2017;9:4015-23. doi:10.1021/acsami.6b11793.
[26] Yadav N, Dubey A, Shukla S, Saini CP, Gupta G, Priyadarshini R, et al. Graphene Oxide-Coated Surface: Inhibition of Bacterial Biofilm Formation due to Specific Surface- Interface Interactions. ACS Omega 2017;2:3070-82. doi:10.1021/acsomega.7b00371.
[27] Mangadlao JD, Santos CM, Felipe MJL, de Leon ACC, Rodrigues DF, Advincula RC. On~the antibacterial mechanism of graphene oxide (GO) Langmuir-Blodgett films. Chem Commun 2015;51:2886-9. doi:10.1039/C4CC07836E.
[28] Henriques PC, Borges I, Pinto AM, Magalhães FD, Gonçalves IC. Fabrication and antimicrobial performance of surfaces integrating graphene-based materials. Carbon N Y 2018;132:709-32. doi:10.1016/j.carbon.2018.02.027.
[29] Jalaja K, Sreehari VS, Kumar PRA, Nirmala RJ. Graphene oxide decorated electrospun gelatin nanofibers: Fabrication, properties and applications. Mater Sci Eng C 2016;64:11-9. doi:10.1016/j.msec.2016.03.036.
[30] Pinto A, Gonçalves C, Sousa D, Ferreira A, Agostinho Moreira J, Gonçalves I, et al. Smaller particle size and higher oxidation improves biocompatibility of graphene-based materials. Carbon N Y 2016;99:318-29. doi:10.1016/j.carbon.2015.11.076.
[31] Gomes RN, Borges I, Pereira AT, Maia AF, Pestana M, Magalhães FD, et al. Antimicrobial graphene nanoplatelets coatings for silicone catheters. Carbon N Y 2018;139:635-47. doi:10.1016/j.carbon.2018.06.044.
[32] Mota C, Puppi D, Dinucci D, Gazzarri M, Chiellini F. Additive manufacturing of star poly(e-caprolactone) wet-spun scaffolds for bone tissue engineering applications. J Bioact Compat Polym 2013;28:320-40. doi:10.1177/0883911513490341.
[33] Dini F, Barsotti G, Puppi D, Coli A, Briganti A, Giannessi E, et al. Tailored star poly (ecaprolactone) wet-spun scaffolds for in vivo regeneration of long bone critical size defects. J Bioact Compat Polym 2016;31:15-30. doi:10.1177/0883911515597928.
[34] Puppi D, Mota C, Gazzarri M, Dinucci D, Gloria A, Myrzabekova M, et al. Additive manufacturing of wet-spun polymeric scaffolds for bone tissue engineering. Biomed Microdevices 2012;14:1115-27. doi:10.1007/s10544-012-9677-0.
[35] Stratton S, Shelke NB, Hoshino K, Rudraiah S, Kumbar SG. Bioactive polymeric scaffolds for tissue engineering. Bioact Mater 2016;1:93-108. doi:10.1016/J.BIOACTMAT.2016.11.001.
[36] Qian Y, Song J, Zhao X, Chen W, Ouyang Y, Yuan W, et al. 3D Fabrication with Integration Molding of a Graphene Oxide/Polycaprolactone Nanoscaffold for Neurite Regeneration and Angiogenesis. Adv Sci 2018;5:1700499.doi:10.1002/advs.201700499.
[37] Azimi B, Nourpanah P, Rabiee M, Arbab S. Poly (e-caprolactone) Fiber: An Overview. J Eng Fiber Fabr 2014;9.
[38] Sousa I, Mendes A, Pereira RF, Bártolo PJ. Collagen surface modified poly(ecaprolactone) scaffolds with improved hydrophilicity and cell adhesion propertie . Mater Lett 2014;134:263-7. doi:10.1016/j.matlet.2014.06.132.
[39] Ege D, Kamali AR, Boccaccini AR. Graphene Oxide/Polymer-Based Biomaterials. Adv Eng Mater 2017;19. doi:10.1002/adem.201700627.
[40] Woodruff MA, Hutmacher DW. The return of a forgotten polymer-Polycaprolactone in the 21st century. Prog Polym Sci 2010;35:1217-56. doi:10.1016/J.PROGPOLYMSCI.2010.04.002.
[41] Sarasam A, Madihally S. Characterization of chitosan-polycaprolactone blends for tissue engineering applications. Biomaterials 2005;26:5500-8. doi:10.1016/j.biomaterials.2005.01.071. doi:10.1021/acsnano.5b02067.
[42] Yang Y, Asiri AM, Tang Z, Du D, Lin Y. Graphene based materials for biomedical applications. Mater Today 2013;16:365-73. doi:10.1016/J.MATTOD.2013.09.004.
[43] Pinto AM, Gonçalves IC, Magalhães FD. Graphene-based materials biocompatibility: A review. Colloids Surfaces B Biointerfaces 2013;111:188-202. doi:10.1016/j.colsurfb.2013.05.022.
[44] Pang L, Dai C, Bi L, Guo Z, Fan J. Biosafety and Antibacterial Ability of Graphene and Graphene Oxide In Vitro and In Vivo. Nanoscale Res Lett 2017;12:564. doi:10.1186/s11671-017-2317-0.
[45] Chen Q, Mangadlao JD, Wallat J, De Leon A, Pokorski JK, Advincula RC. 3D printing biocompatible polyurethane/poly(lactic acid)/graphene oxide nanocomposites: Anisotropic properties. ACS Appl Mater Interfaces 2017;9:4015-23. doi:10.1021/acsami.6b11793.
[46] Yadav N, Dubey A, Shukla S, Saini CP, Gupta G, Priyadarshini R, et al. Graphene Oxide-Coated Surface: Inhibition of Bacterial Biofilm Formation due to Specific Surface- Interface Interactions. ACS Omega 2017;2:3070-82. doi:10.1021/acsomega.7b00371.
[47] Mangadlao JD, Santos CM, Felipe MJL, de Leon ACC, Rodrigues DF, Advincula RC. On the antibacterial mechanism of graphene oxide (GO) Langmuir-Blodgett films. Chem Commun 2015;51:2886-9. doi:10.1039/C4CC07836E.
[48] Stratton S, Shelke NB, Hoshino K, Rudraiah S, Kumbar SG. Bioactive polymeric scaffolds for tissue engineering. Bioact Mater 2016;1:93-108. doi:10.1016/J.BIOACTMAT.2016.11.001.
[49] Qian Y, Song J, Zhao X, Chen W, Ouyang Y, Yuan W, et al. 3D Fabrication with Integration Molding of a Graphene Oxide/Polycaprolactone Nanoscaffold for Neurite Regeneration and Angiogenesis. Adv Sci 2018;5:1700499. doi:10.1002/advs.201700499.
[50] Azimi B, Nourpanah P, Rabiee M, Arbab S. Poly (e-caprolactone) Fiber: An Overview. J Eng Fiber Fabr 2014;9.
[51] Wei X, Li D, Jiang W, Gu Z, Wang X, Zhang Z, et al. 3D Printable Graphene Composite. Sci Rep 2015;5:1-7. doi:10.1038/srep11181.
[52] Hummers WS, Offeman RE. Preparation of Graphitic Oxide. J Am Chem Soc 1958;80:1339. doi:10.1021/ja01539a017.
[53] Neves SC, Mota C, Longoni A, Barrias CC, Granja PL, Moroni L. Additive manufactured polymeric 3D scaffolds with tailored surface topography influence mesenchymal stromal cells activity. Biofabrication 2016;8:1-15. doi:10.1088/1758-5090/8/2/025012.

## Claims

1. Method for constructing a fibrous scaffold of Poly(e-caprolactone) and Graphene Oxide by additive manufacturing and wet-spinning of comprising the steps of:
a) Dispersing graphene oxide (GO) in Tetrahydrofuran (THF), solvent at a final concentration of 5% w/w to 7.5% w/w;
b) Dissolving Poly(e-caprolactone) - PLC- in the previous mixture at a concentration of 7.5% to 15% w/v (weight of PCL per volume of solvent);
c) Loading the previous mixture into a syringe or a 3D printer head
d) Extruding the THF/PCL/GO mixture into a coagulation bath consisting of ethanol
e) Rinsing PCL/GO scaffolds with ethanol and drying the scaffolds.

2. Method according to claim 1 wherein in step a) graphene oxide (GO) is dispersed in Tetrahydrofuran (THF), solvent at a final concentration of 5% w/w and in step b) Poly(e-caprolactone) - PLC- is dissolved in the previous mixture at a concentration of 7.5% w/v.

3. Method according to claims 1 or 2 wherein the said extrusion of THF/PCL/GO mixture is performed layer-by-layer in a 3D plotting machine or a 3D printer.

## Patentansprüche

1. Verfahren zur Herstellung eines faserigen Gerüsts aus Poly(e-caprolacton) und Graphenoxid durch additives Spinnen und Nassspinnen umfassend die Schritte:
a) Dispergieren von Grahenoxid (GO) in Tetrahydrofuran (THF), Lösungsmittel mit einer Endkonzentration von 5 Gew.-% bis 7,5 Gew.-%;
b) Lösen von Poly(e-caprolacton) - PLC - in der vorherigen Mischung in einer Konzentration von 7,5 bis 15 Gew.-% (PCL-Gewicht pro Volumen des Lösungsmittels);
c) Laden der vorherigen Mischung in eine Spritze oder einen 3D-Druckerkopf;
d) Extrudieren der THF/PCL/GO-Mischung in ein Koagulationsbad aus Ethanol;
e) Spülen der PCL/GO-Gerüste mit Ethanol und Trocknen der Gerüste.

2. Verfahren gemäß Anspruch 1, wobei in Schritt a) Graphenoxid (GO) in Tetrahydrofuran (THF) dispergiert wird, Lösungsmittel in einer Endkonzentration von 5 Gew.-%, und in Schritt b) Poly(e-caprolactone) - PLC - in die vorherige Mischung in einer Konzentration von 7,5 Gew.-% gelöst wird.

3. Verfahren gemäß Ansprüche 1 oder 2, wobei genanntes Extrudieren der THF/PCL/GO-Mischung schichtweise in einer 3D-Plottmaschine oder einem 3D-Drucker erfolgt.

## Revendications

1. Méthode de construction d'un échafaudage fibreux de poly(e-caprolactone) et d'Oxyde de Graphène par fabrication additive et filage humide, comprenant les étapes suivantes :
a) Dispersion d'oxyde de graphène (GO) dans du tétrahydrofuranne (THF), un solvant d'une concentration finale comprise entre 5 % p/p et 7,5 % p/p ;
b) Dissolution du poly(e-caprolactone) - PLC- dans le mélange précédent à une concentration comprise entre 7,5 % et 15 % p/v (poids du PLC par volume de solvant) ;
c) Chargement du mélange précédent dans une seringue ou une tête d'impression 3D
d) Extrusion du mélange de THF/PCL/GO dans un bain de coagulation composé d'éthanol
e) Rinçage des échafaudages de PCL/GO avec de l'éthanol et séchage des échafaudages.

2. Méthode selon la revendication 1 où à l'étape a) l'oxyde de graphène (GO) est dispersé dans le tétrahydrofuranne (THF), un solvant d'une concentration finale de 5 % p/p et à l'étape b) le poly(e-caprolactone) - PLC - est dissout dans le mélange antérieur d'une concentration de 7,5 % p/v.

3. Méthode selon les revendications 1 ou 2 où ladite extrusion du mélange de THF/PCL/GO est réalisée couche par couche dans une machine de traçage 3D ou une imprimante 3D.
